(19) Europäisches Patentamt
European Patent Office
Office européen des brevets

(11) **EP 4 202 526 A1**

(12) **EUROPEAN PATENT APPLICATION**
published in accordance with Art. 153(4) EPC

(43) Date of publication:
**28.06.2023 Bulletin 2023/26**

(21) Application number: **21857492.9**

(22) Date of filing: **30.07.2021**

(51) International Patent Classification (IPC):
**G02B 23/24** (2006.01)     **A61B 1/00** (2006.01)

(86) International application number:
**PCT/CN2021/109859**

(87) International publication number:
**WO 2022/037397 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2020 CN 202010852343**

(71) Applicant: **Shanghai Micromission Medical Co., Ltd.**
**Shanghai 201203 (CN)**

(72) Inventors:
• **LIANG, Xiangnan**
  **Shanghai 201203 (CN)**
• **LI, Qian**
  **Shanghai 201203 (CN)**
• **HE, Yuyuan**
  **Shanghai 201203 (CN)**
• **HE, Chao**
  **Shanghai 201203 (CN)**
• **CAO, Lun**
  **Shanghai 201203 (CN)**

(74) Representative: **Patentship**
**Patentanwaltsgesellschaft mbH**
**Elsenheimerstraße 65**
**80687 München (DE)**

(54) **ENDOSCOPE SYSTEM AND METHOD FOR DETECTING WHEN TAIL END OF ENDOSCOPE COMES INTO CONTACT WITH TISSUE**

(57) The present invention provides an endoscope system and a method for detecting the contact between the tail end of the endoscope and the tissue, in which the video stream gain and an exposure time can be adjusted to maintain a current brightness of a video stream within the target brightness range, and the distance from the end of the endoscope to the target tissue can be measured. When the distance from the end of the endoscope to the target tissue is smaller than a predetermined distance, a contact alert is provided, and contact determination is triggered. During detection of a rate of change of a ratio of the current brightness to an exposure amount is smaller than a threshold, texture feature information of the target tissue may be extracted respectively before and after a power of output light is increased and compared to determine whether the end of the endoscope is in contact with the target tissue. By determining whether the end of the endoscope is in contact with the target tissue through comparing the texture feature information of the target tissue extracted respectively before and after the power of the output light is increased to find whether there is a texture feature change, the endoscope system of the present invention can provide a more accurate contact indication and effectively avoid the endoscope from causing damage to the target tissue during its examination of the tissue.

Fig. 1

## Description

## TECHNICAL FIELD

The present invention relates to the field of medical devices and, in particular, to an endoscope system and a method for detecting contact of an end of an endoscope with tissue.

## BACKGROUND

As medical technology continues to advance, endoscopes are finding increasingly widespread use as detectors integrating traditional optics, ergonomics, precision machinery, modern electronics, mathematics and software. An endoscope can be delivered into the body of a subject (e.g., into the esophagus thereof) in order to take an image of a target site, from which, it can be determined if there is a pathological change at the target site. Endoscopes are very useful for physicians because they can visualize pathological changes that cannot be visualized by X-rays. For example, with the aid of an endoscope, a physician can observe an ulcer or a tumor in the stomach and make the best treatment plan based on the observation.

During use of an endoscope for examination, there is a risk that it (or its end) may approach too close to, or inadvertently come into contact with, tissue at a target site. This could interfere with the examination, or even cause damage to the tissue. Conventionally, control of optical power of automatic exposure and an illuminating light source is mainly relied for detecting whether an endoscope is in contact with tissue. However, due to the translucent nature of human tissue, this approach is prone to misdetermination. Therefore, there is a need for an endoscope system combining multiple methods for detecting whether an endoscope is about to or has come into contact with tissue.

## SUMMARY OF THE INVENTION

It is an object of the present invention to provide an endoscope system and a method for detecting contact of an end of an endoscope with tissue, which can provide a contact alert and a contact indication, which are helpful in avoiding damage to the tissue, during examination of the tissue by the endoscope.

Accordingly, the present invention provides an endoscope system, which comprises an endoscope, an illumination module, an endoscope drive module, an endoscope control module and a contact detection module.

The illumination module is configured to provide an output light for illuminating a target tissue and to generate a reflected light.

The endoscope drive module is configured to acquire a scene of the target tissue through capturing the reflected light based on an exposure time and a video stream gain and to output the scene in a form of a video stream.

The endoscope control module is communicatively connected to the endoscope drive module and configured to acquire a current brightness of the video stream from the video stream, and wherein a video stream gain and an exposure time are adjusted, such that the current brightness of the video stream is within a target brightness range.

The contact detection module is communicatively connected to each of the endoscope control module and the illumination module, and comprises a contact determination unit. When the current brightness of the video stream lies within the target brightness range and a distance from an end of the endoscope to the target tissue is smaller than a predetermined distance, the contact determination unit is configured to determine a relationship between a rate of change of a ratio of the current brightness to an exposure amount and a threshold. When the rate of change is smaller than the threshold, the contact determination unit is configured to control the illumination module to increase the power of the output light, and then extract and compare texture feature information of the target tissue respectively before and after the power of the output light is increased so as to determine whether the end of the endoscope is in contact with the target tissue, and to provide a contact indication.

Optionally, the contact detection module may further comprise a ranging unit configured to detect the distance from the end of the endoscope to the target tissue.

Optionally, the ranging unit may provide a contact alert upon detection of the distance from the end of the endoscope to the target tissue is smaller than the predetermined distance.

Optionally, the endoscope may be a three-dimensional endoscope, wherein the ranging unit obtains the distance from the end of the endoscope to the target tissue using a ranging method based on a binocular stereoscopic imaging.

Optionally, the endoscope control module may comprise a brightness acquisition unit configured to acquire the current brightness of the video stream.

Optionally, in case of the video stream being a YUV-coded stream, the brightness acquisition unit may be configured to take an averaged or a weighted value of Y values of all or some pixels in an image of a current frame as the current brightness.

Optionally, in case of the video stream being a RGB-coded stream, the brightness acquisition unit may be configured to derive a brightness of each pixel in the image of the current frame from a RGB value of the pixel and then take an averaged or a weighted value of the brightness of all or some of the pixels in the image as the current brightness. Alternatively, the brightness acquisition unit may be configured to take an averaged or a weighted value of G values of all or some of the pixels in the image of the current frame as the current brightness.

Optionally, the endoscope control module may comprise an exposure control unit configured to determine whether the current brightness of the video stream is within the target brightness range and, when the current brightness of the video stream is not within the target brightness range, the exposure control unit is configured to adjust the video stream gain and the exposure time to enable the current brightness of the video stream to be within the target brightness range.

Optionally, when the current brightness is lower than a lower limit of the target brightness range, the exposure control unit may adjust the current brightness by increasing the exposure amount.

Optionally, the exposure control unit may determine whether a maximum exposure time and a minimum video stream gain satisfy an exposure amount requirement of the target brightness range, and

> if the requirement is satisfied, on a basis of the minimum video stream gain, adjusting the exposure time to enable the current brightness to be within the target brightness range; or
> if the requirement is not satisfied, increasing the video stream gain and determining whether the maximum exposure time and a maximum video stream gain satisfy the exposure amount requirement of the target brightness range, and if the requirement is satisfied, on a basis of the maximum exposure time, adjusting the video stream gain to enable the current brightness of the video stream to be within the target brightness range.

Optionally, when the current brightness is higher than an upper limit of the target brightness range, the exposure control unit may adjust the current brightness by reducing the exposure amount.

Optionally, the exposure control unit may determine whether a maximum exposure time and a minimum video stream gain satisfy an exposure amount requirement of the target brightness range,

> if the requirement is satisfied, on a basis of the maximum exposure time, adjusting the video stream gain to enable the current brightness of the video stream to be within the target brightness range; or
> if the requirement is not satisfied, reducing the exposure time and determining whether a minimum exposure time and the minimum video stream gain satisfy the exposure amount requirement of the target brightness range, and if the requirement is satisfied, on a basis of the minimum video stream gain, adjusting the exposure time to enable the current brightness of the video stream to be within the target brightness range.

Optionally, when the exposure control unit is not able to adjust the video stream gain and the exposure time to enable the current brightness of the video stream to be within the target brightness range, the illumination module may be controlled to adjust the power of the output light.

Optionally, the contact detection module may further comprise an illumination module adjusting unit communicatively connected to each of the illumination module and the exposure control unit. Additionally, the illumination module adjusting unit may be configured to determine whether the current brightness is within the target brightness range based on the video stream gain and the exposure time output from the exposure control unit, and to adjust the power of the output light through the illumination module.

Optionally, the illumination module adjusting unit may be also communicatively connected to the contact determination unit, further configured to, under a control of the contact determination unit, adjust the power of the output light through the illumination module, and while the contact determination unit is determining whether the end of the endoscope is in contact with the target tissue, the illumination module adjusting unit is controlled not to be instructed by the exposure control unit to adjust the power of the output light any more.

Optionally, the endoscope drive module may comprise a solid-state photosensitive unit and a scene drive unit, the solid-state photosensitive unit configured to receive the reflected light that carries information of the scene of the target tissue, and to perform optical-to-electronic conversion on the information of the scene of the target tissue and to output it as the video stream, wherein the scene drive unit is configured to control the solid-state photosensitive unit based on the video stream gain and the exposure time received from the endoscope control module.

Optionally, the contact detection module may further comprise an illumination module adjusting unit communicatively connected to each of the contact determination unit and the exposure control unit, the illumination module adjusting unit configured to receive the video stream gain, the exposure time and the current brightness output from the exposure control unit, wherein the contact determination unit is configured to determine the rate of change of the ratio of the current brightness to the exposure amount based on the video stream gain, the exposure time and the current brightness received by the illumination module adjusting unit.

Optionally, the contact determination unit may comprise a texture feature extraction element and a texture feature comparison element, which are configured to extract and compare texture features of the target tissue, respectively.

Optionally, the endoscope system may further comprise a video pipeline and a central controller, the central controller comprising a video processing unit, wherein the video stream output from the contact detection module is transmitted to the video pipeline, and wherein the video processing unit processes the video stream in the video pipeline and transmits the processed video stream to a display device for display thereon.

The present invention further provides a corresponding method for detecting a contact of an end of an endoscope with tissue, which comprises:

providing an output light for illuminating a target tissue to generate a reflected light;
acquiring a scene of the target tissue through capturing the reflected light based on an exposure time and a video stream gain and outputting the scene in a form of a video stream;
acquiring a current brightness of the video stream, and adjusting the video stream gain and the exposure time to enable the current brightness of the video stream to be within a predetermined target brightness range; and
when the current brightness of the video stream is within the target brightness range and a distance from an end of the endoscope to the target tissue is smaller than a predetermined distance,
determining whether a rate of change of a ratio of the current brightness to an exposure amount is smaller than a threshold, if so, then
extracting a texture feature information of the target tissue as a first texture feature information;
increasing a power of the output light and then extracting a texture feature information of the target tissue as a second texture feature information; and
comparing the first texture feature information and the second texture feature information, and if no texture feature change is identified, determining that a contact of the end of the endoscope with the target tissue has occurred.

Optionally, the distance from the end of the endoscope to the target tissue may be obtained by a ranging method based on binocular stereoscopic imaging.

Optionally, when the current brightness is lower than a lower limit of the target brightness range, the current brightness may be adjusted by increasing the exposure amount.

Optionally, it may be determined whether a maximum exposure time and a minimum video stream gain satisfy an exposure amount requirement of the target brightness range, and
if the requirement is satisfied, on a basis of the minimum video stream gain, the exposure time may be adjusted to enable the current brightness to be within the target brightness range.

Otherwise, if the requirement is not satisfied, the video stream gain may be increased, followed by determining whether the maximum exposure time and a maximum video stream gain satisfy the exposure amount requirement of the target brightness range, and if the requirement is satisfied, on a basis of the maximum exposure time, the video stream gain may be adjusted to enable the current brightness of the video stream to be within the target brightness range.

Optionally, before it is determined whether the rate of change of the ratio of the current brightness to the exposure amount is smaller than the threshold, if the maximum exposure time and the maximum video stream gain do not satisfy the exposure amount requirement of the target brightness range, then the power of the output light may be adjusted.

Optionally, if the current brightness is higher than an upper limit of the target brightness range, the current brightness may be adjusted by reducing the exposure amount.

Optionally, it may be determined whether a maximum exposure time and a minimum video stream gain satisfy an exposure amount requirement of the target brightness range, and
if the requirement is satisfied, on a basis of the maximum exposure time, the video stream gain may be adjusted to enable the current brightness to be within the target brightness range.

Otherwise, if the requirement is not satisfied, the exposure time may be reduced, followed by determining whether a minimum exposure time and the minimum video stream gain satisfy the exposure amount requirement of the target brightness range, and if the requirement is satisfied, on a basis of the minimum video stream gain, the exposure time may be adjusted to enable the current brightness of the video stream to be within the target brightness range.

Optionally, before it is determined whether the rate of change of the ratio of the current brightness to the exposure amount is smaller than the threshold, if the minimum exposure time and the minimum video stream gain do not satisfy the exposure amount requirement of the target brightness range, the power of the output light may be adjusted.

Optionally, when the distance from the end of the endoscope to the target tissue is smaller than the predetermined distance, a contact alert may be provided.

In summary, the present invention provides an endoscope system and a method for detecting a contact of an end of an endoscope with target tissue, in which a video stream gain and an exposure time can be adjusted to maintain a current brightness of a video stream within a target brightness range, and the distance from the end of the endoscope to the target tissue can be measured. When the distance from the end of the endoscope to the target tissue is smaller than a predetermined distance, a contact alert is provided, and contact determination is triggered. During detection of a rate of change of a ratio of the current brightness to an exposure amount is smaller than a threshold, texture feature information of the target tissue may be extracted respectively before and after a power of output light is increased and compared to determine whether the end of the endoscope is in contact with the target tissue. By determining whether the end of the endoscope is in contact with the target tissue through comparing the texture feature information of the target tissue extracted respectively before and after the power of the output light is increased to find whether there is a texture feature change, the endoscope system of the present invention can provide a more accurate contact indication and effectively avoid the endoscope from causing damage to the target tissue during its examination of the tissue.

## BRIEF DESCRIPTION OF THE DRAWINGS

Fig. 1 is a diagram depicting the structure of a part of an endoscope system according to an embodiment of the present invention and data stream control therein.

Fig. 2 is a flowchart of a process for tuning a current brightness of a video stream into a target brightness range according to an embodiment of the present invention.

Fig. 3 is a flowchart of a process for tuning the current brightness into the target brightness range through adjusting an exposure amount according to an embodiment of the present invention.

Fig. 4 schematically illustrates how a distance is measured using binocular stereoscopic imaging.

Fig. 5 is a diagram schematically illustrating a relationship between a distance L from an end of an endoscope to tissue and a parallax d.

Fig. 6 schematically illustrates how a parallax is derived from "left-eye" and "right-eye" images.

Fig. 7 explains operation of a contact detection module in an endoscope system according to an embodiment of the present invention.

Fig. 8 is a schematic diagram showing a relationship between an exposure amount and a distance from an end of an endoscope to tissue.

Fig. 9 is a schematic diagram corresponding to Fig. 8, showing a relationship between a current brightness and the distance from the end of the endoscope to the tissue.

Fig. 10 is a schematic diagram showing a relationship between a ratio of the current brightness to the exposure amount and the distance from the end of the endoscope to the tissue.

Fig. 11 is a schematic diagram showing a relationship between a rate of change of the ratio of the current brightness to the exposure amount and the distance from the end of the endoscope to the tissue.

In these figures,
100, target tissue; 200, an endoscope; 210, an illumination module; 211, a main light source; 212, an illumination controller; 220, an endoscope drive module; 221, a scene drive unit; 222, a solid-state photosensitive unit; 230, an endoscope control module; 231, a brightness acquisition unit; 232, an exposure control unit; 240, a contact detection module; 241, a ranging unit; 242, a contact determination module; 243, an illumination module adjusting unit; 242a, a texture feature extraction element; 242b, a texture feature comparison element; 250, a video pipeline; 260, a central controller; 261, a user control logic; 262, a user interface; 263, a video processing unit; and 300, a display.

## DETAILED DESCRIPTION

The proposed endoscope system and method will be described in greater detail below with reference to specific embodiments and to the accompanying drawings. Advantages and features of the invention will become more apparent from the following description and the annexed figures. However, it is to be noted that the concept of the present invention can be embodied in various forms and is not limited to the particular examples disclosed herein. The figures are provided in a very simplified form not necessarily presented to scale and only intended to help in explaining the disclosed embodiments in a convenient and clear way.

As used herein, the terms "first", "second" and the like are meant to distinguish similar elements from each other rather than necessarily indicate specific sequential or temporal orderings. It is to be understood that the terms so used are interchangeable under appropriate circumstances such that the embodiments of the invention described herein are, for example, capable of operation in other orientations than those illustrated or otherwise described herein. Likewise, if any method described herein includes a sequence of steps, then the order of the steps presented herein does not have to be the only order in which the steps may be performed, and some of the steps may be omitted, and/or some other steps not described herein may be added to the method. If any element in an embodiment of the present invention depicted in one of the accompanying drawings is identical to that in another one or other ones of the drawings, for the sake of clarity, a reference numeral for the element may not be marked in all of these figures even if the element is readily identifiable therein.

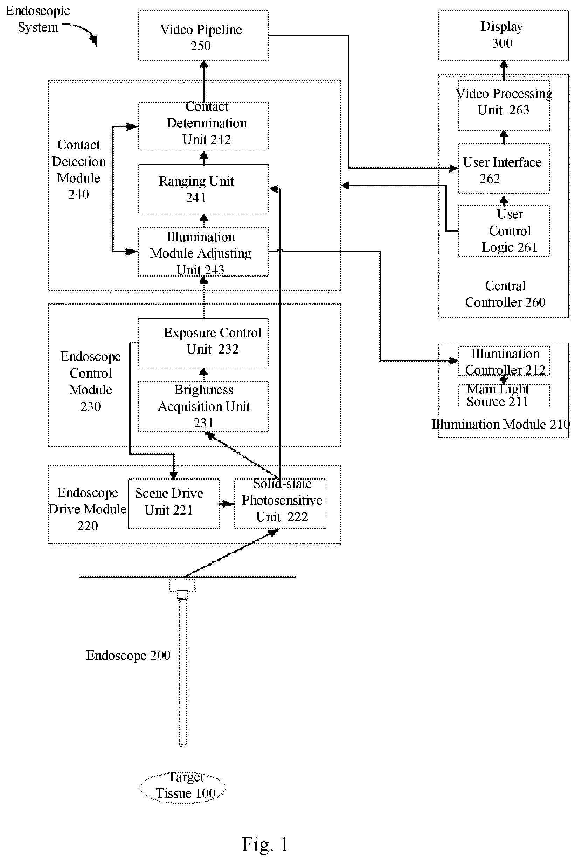

Fig. 1 is a diagram depicting the structure of a part of an endoscope system according to an embodiment of the present invention and data stream control therein. As shown in Fig. 1, the endoscope system of this embodiment includes an endoscope 200, an illumination module 210, an endoscope drive module 220, an endoscope control module 230 and a contact detection module 240.

The illumination module 210 is configured to provide the endoscope system with output light (e.g., visible light) with a predetermined power for illuminating target tissue 100 to generate reflected light.

The endoscope drive module 220 is configured to acquire a scene of the tissue through capturing the reflected light based on an exposure time T and a video stream gain G and to output the scene in the form of a video stream.

The endoscope control module 230 is communicatively connected to the endoscope drive module 220 and configured to acquire a current brightness B of the video stream from the video stream and tune the current brightness B of the video stream into a predetermined target brightness range by adjusting the video stream gain G and the exposure time T.

The contact detection module 240 is communicatively connected to both the endoscope control module 230 and the illumination module 210 and includes a contact determination unit 242. When the current brightness B of the video stream lies within the target brightness range, and when a distance from an end of the endoscope 200 to the tissue 100 is smaller than a predetermined distance, the contact determination unit is configured to determine a relationship between a rate of change of a ratio of the current brightness B to an exposure amount (which is equal to the exposure time T times the video stream gain G) and a predetermined threshold. When the rate of change of the ratio of the current brightness B to the exposure amount is smaller than the threshold, the illumination module 210 is controlled to increase the power of the output light, and pieces of texture feature information of the target tissue are then extracted respectively before and after the power of the output light is increased and compared to determine whether they are identical and hence whether the end of the endoscope 200 is in contact with the target tissue 100.

Specifically, in this embodiment, the illumination module includes a main light source 211 and an illumination controller 212 for controlling the main light source. This embodiment is limited neither to any particular location of the illumination module nor to any particular form of the light source of the illumination module. For example, light output from the main light source 211 may be transmitted to the end of the endoscope 200 by a connector accommodated in an illumination channel of the endoscope 200 and thereby reaches the target tissue 100. The connector may be an optical fiber, for example. The illumination module may also be disposed in the illumination channel of the endoscope 200. For example, the main light source 211 may be implemented as an LED lamp and the illumination controller 212 as an LED drive circuit, and both the LED lamp and the LED drive circuit may be provided in the illumination channel.

The endoscope drive module 220 includes a solid-state photosensitive unit 222 and a scene drive unit 221. The solid-state photosensitive unit 222 is configured to receive the reflected light that carries information of the scene of the target tissue, perform optical-to-electronic conversion on the information of the scene of the target tissue and output it in the form of a video stream. Additionally, the solid-state photosensitive unit 222 is coupled to the brightness acquisition unit 231 as detailed below, to output the video stream. The scene drive unit 221 is configured to control the solid-state photosensitive unit 222 by providing it with configuration parameters such as exposure time, video stream gain, etc. Further, the scene drive unit 221 receives output parameters including the exposure time T and the video stream gain G from the exposure control unit 232 as detailed below to control the solid-state photosensitive unit 222 to receive the reflected light and perform the optical-to-electronic conversion. In this embodiment, the endoscope is a three-dimensional endoscope. Accordingly, the solid-state photosensitive unit 222 includes a left solid-state photosensitive element and a right solid-state photosensitive element, and the scene drive unit 221 includes a left scene drive element corresponding to the left solid-state photosensitive element and a right scene drive element corresponding to the right solid-state photosensitive element. In this embodiment, the solid-state photosensitive unit 222 may be a complementary metal oxide semiconductor (CMOS) or charge coupled device (CCD) sensor.

Referring to Fig. 1, the endoscope control module 230 includes a brightness acquisition unit 231 and an exposure control unit 232. The brightness acquisition unit 231 is coupled to the solid-state photosensitive unit 222, and the exposure control unit 232 is coupled to the brightness acquisition unit 231, the scene drive unit 221 and the illumination module adjusting unit 243 as detailed below. The brightness acquisition unit 231 receives the video stream from the solid-state photosensitive unit 222, acquires a brightness B of an image of the current frame in the video stream in real time as the current brightness, and transmits the current brightness B to the exposure control unit 232. The exposure control unit 232 is configured to adjust an exposure amount (an exposure time and/or a video stream gain) of the solid-state photosensitive unit 222 based on the predetermined target brightness range and the received current brightness B and to output the adjusted exposure amount to the scene drive unit 221 and the illumination module adjusting unit 243, so as to enable the current brightness B of the video stream to be within the predetermined target brightness range. Those skilled in the art would appreciate that the current frame in the video stream changes dynamically, that is, the image of the current frame varies over time. Thus, by the phrase "so as to enable the current brightness B of the video stream to bewithin the predetermined target brightness range", it is meant that the solid-state photosensitive unit 222 is adjusted based on the exposure time T and the video stream gain G derived from the image of the current frame so that a brightness B of an image of a subsequent frame lies in the predetermined target brightness range at a later time when the subsequent frame becomes the current frame.

The embodiment is not limited to any particular method of acquiring the current brightness B. For example, when the video stream output from the solid-state photosensitive unit 222 is a YUV-coded stream, the brightness acquisition unit 231 may take an averaged or weighted value of Y values of all or some pixels in the image as the current brightness B. As another example, when the video stream output from the solid-state photosensitive unit 222 is an RGB-coded stream, the brightness acquisition unit 231 may derive brightness of the pixels in the image from their RGB values and then take an averaged or weighted value of the brightness of all or some of the pixels as the current brightness B. The derivation of the brightness of the pixels from their RGB values may be accomplished by simply taking the G values that are more sensitive to the human eye, or values obtained by weighting the R, G and B values, for example, according to $Y=0.2126*R + 0.7152*G + 0.0722*B$ or $Y=0.299*R + 0.587*G + 0.114*B$, as the brightness.

In this embodiment, the endoscope is a three-dimensional endoscope. Accordingly, the exposure control unit 232 includes a first control loop and a second control loop. The first and second control loops are both configured for video stream gain control and exposure time control. The first control loop controls a gain of a left video stream and/or an exposure time of the left solid-state photosensitive element, and the second control loop controls a gain of a right video stream and/or an exposure time of the right solid-state photosensitive element.

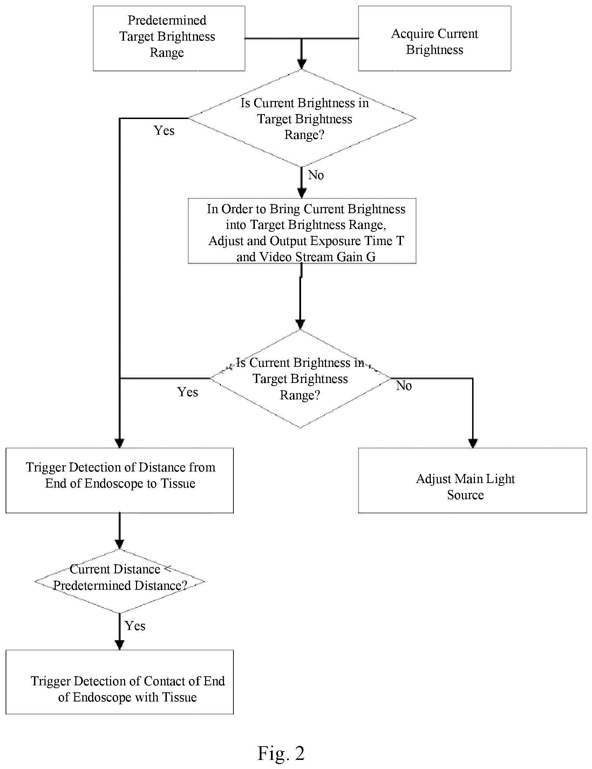

Fig. 2 discloses a method of tuning the current brightness B of the video stream into the target brightness range. Here, a lower limit of the target brightness range is denoted as $B_{min}$, and an upper limit of the target brightness range as $B_{max}$. The exposure control unit 232 receives, from the brightness acquisition unit 231, the current brightness B of the image of the current frame in the video stream. Moreover, the exposure control unit 232 determines whether the current brightness lies in the predetermined target brightness range $(B_{min}, B_{max})$. If the current brightness B is in the predetermined target brightness range $(B_{min}, B_{max})$, the exposure time T and the video stream gain G are output to the illumination module adjusting unit 243, and optionally to the scene drive unit 221. If the current brightness B is outside the predetermined target brightness range $(B_{min}, B_{max})$, the exposure amount is adjusted to bring the current brightness B into the predetermined target brightness range $(B_{min}, B_{max})$. If it is impossible to shift the current brightness B into the predetermined target brightness range $(B_{min}, B_{max})$ through adjusting the exposure time T and the video stream gain G, the exposure control unit 232 feeds back this information to the illumination module adjusting unit 243 coupled to the illumination module 210, which then adjusts the power of the output light to create a change in the current brightness of the target tissue, i.e., current brightness of the video stream.

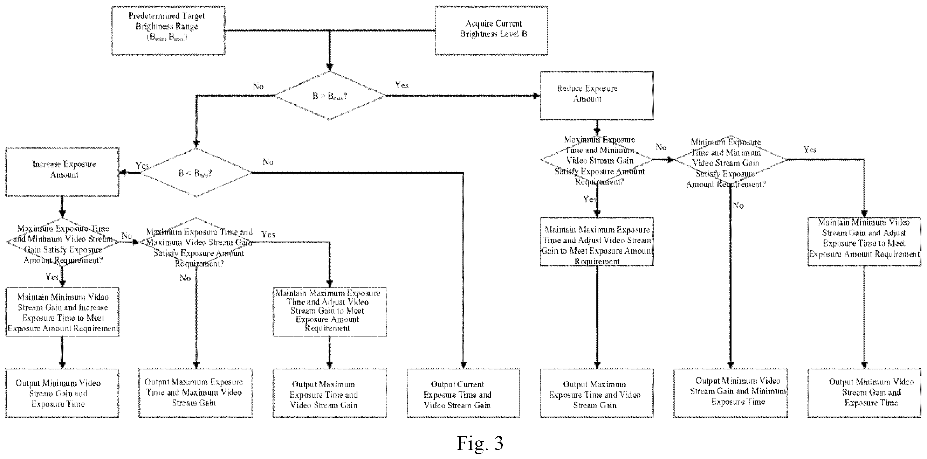

Fig. 3 shows a method of tuning the current brightness B into the predetermined target brightness range $(B_{min}, B_{max})$ through adjusting the exposure time T and the video stream gain G. The method will be detailed below with reference to Fig. 3.

On the one hand, when the current brightness B of the scene of the tissue is lower than the lower limit $B_{min}$ of the target brightness range, the exposure control unit 232 increases the exposure amount to adjust the current brightness B. The exposure time T is first increased, and it is then determined whether a maximum exposure time $T_{max}$ and a minimum video stream gain $G_{min}$ satisfy an exposure amount requirement of the target brightness range.

If the requirement is satisfied, on the basis of the minimum video stream gain $G_{min}$, the exposure time T is adjusted to tune the current brightness B of the video stream into the target brightness range $(B_{min}, B_{max})$, and both the minimum video stream gain $G_{min}$ and the adjusted exposure time T are output to the scene drive unit 221 and the illumination module adjusting unit 243 as detailed below.

If the requirement is not satisfied, the video stream gain G is increased, and it is then determined whether the maximum exposure time $T_{max}$ and a maximum video stream gain $G_{max}$ satisfy the exposure amount requirement of the target brightness range. If the requirement is not satisfied, the current exposure time T and video stream gain G are maintained, and the exposure control unit 232 feeds information indicating that the current brightness is still outside the target brightness range (e.g., the information contains the maximum exposure time $T_{max}$ and the maximum video stream gain $G_{max}$) back to the illumination module adjusting unit 243 coupled to the illumination module 210. If the requirement is satisfied, on the basis of the maximum exposure time $T_{max}$, the video stream gain G is adjusted to tune the current brightness B of the video stream into the target brightness range $(B_{min}, B_{max})$, and both the maximum exposure time $T_{max}$ and the adjusted video stream gain G are output to the scene drive unit 221 and the illumination module adjusting unit 243 as detailed below.

On the other hand, when the current brightness B is higher than the upper limit $B_{max}$ of the target brightness range, the exposure control unit 232 reduces the exposure amount to adjust the current brightness B. Specifically, the video stream gain G is first reduced, and it is then determined whether the maximum exposure time $T_{max}$ and the minimum video stream gain $G_{min}$ satisfy an exposure amount requirement of the target brightness range. If the requirement is satisfied, on the basis of the maximum exposure time $T_{max}$, the video stream gain G is adjusted to tune the current brightness B of the video stream into the target brightness range $(B_{min}, B_{max})$, and both the maximum exposure time T max and the adjusted video stream gain G are output to the scene drive unit 221 and the illumination module adjusting unit 243 as detailed below.

If the requirement is not satisfied, the exposure time T is increased, and it is then determined whether a minimum exposure time $T_{min}$ and the minimum video stream gain $G_{min}$ satisfy the exposure amount requirement of the target brightness range. If the requirement is not satisfied, the current exposure time T and video stream gain G are maintained, and the exposure control unit 232 feeds information indicating that the current brightness is still outside the target brightness range (e.g., the information contains the minimum exposure time $T_{min}$ and the minimum video stream gain $G_{min}$) back

to the illumination module adjusting unit 243 coupled to the illumination module 210. If the requirement is satisfied, on the basis of the minimum video stream gain $G_{min}$, the exposure time T is adjusted to tune the current brightness B of the video stream into the target brightness range $(B_{min}, B_{max})$, and both the minimum video stream gain $G_{min}$ and the adjusted exposure time T are output to the scene drive unit 221 and the illumination module adjusting unit 243 as detailed below.

In other words, in this embodiment, the exposure control unit 232 tunes the current brightness of the video stream into the target brightness range by adjusting the video stream gain G or the exposure time T and outputs the adjusted video stream gain G or exposure time T to the scene drive unit 221 and the illumination module adjusting unit 243 as detailed below. However, in alternative embodiments of the present invention, the current brightness of the scene of the tissue may also be adjusted otherwise.

With continued reference to Fig. 2, when the current brightness of the video stream lies within the target brightness range, i.e., when the product TG of the video stream gain G and the exposure time T output from the exposure control unit 232 is greater than or equal to $(TG)_{min}$ and smaller than or equal to $(TG)_{max}$, detection of the distance between the end of the endoscope 200 and the tissue is triggered. Here, TG represents the product of the output video stream gain G and the exposure time T; $(TG)_{min}$ is the product of the minimum exposure time $T_{min}$ and the minimum video stream gain $G_{min}$; and $(TG)_{max}$ is the product of the maximum exposure time $T_{max}$ and the maximum video stream gain $G_{max}$. When the detected current distance between the end of the endoscope 200 and the tissue is smaller than a predetermined distance $L_{th}$, determination of whether the end of the endoscope 200 is in contact with the tissue is triggered. On the other hand, when the current brightness of the video stream is still outside the target brightness range, the illumination module adjusting unit 243 receives the information indicating that the current brightness is outside the target brightness range and adjusts the main light source 211 through the illumination controller 212. In a preferred embodiment, the information indicating that the current brightness is outside the target brightness range contains both the maximum exposure time $T_{max}$ and the maximum video stream gain $G_{max}$ output from the exposure control unit 232, or both the minimum exposure time $T_{min}$ and the minimum video stream gain $G_{min}$ output from the exposure control unit 232. The exposure control unit 232 and the illumination module adjusting unit may incrementally adjust the exposure amount and the power of the main light source 211, respectively, until the current brightness B of the video stream is shifted into the target brightness range $(B_{min}, B_{max})$.

The contact detection module 240 further includes a ranging unit 241, in addition to the contact determination unit 242 and the illumination module adjusting unit 243. The illumination module adjusting unit is configured to control the output power of the main light source 211 through the illumination controller 212. The ranging unit 241 is configured to detect the distance between the end of the endoscope 200 and the target tissue 100. The contact determination unit 242 is configured to determine whether the end of the endoscope 200 is in contact with the target tissue 100.

In this embodiment, the illumination module adjusting unit 243 is coupled to each of the exposure control unit 232, the contact determination unit 242 and the illumination controller 212. As shown in Fig. 2, the illumination module adjusting unit 243 receives the exposure time T and the video stream gain G output from the exposure control unit 232 and determines whether the exposure time T and the video stream gain G output from the exposure control unit 232 correspond to either of the limit values $(TG)_{min}$ and $(TG)_{max}$. If so, it is known that it is impossible for the exposure control unit 232 to bring the current brightness B into the target brightness range $(B_{min}, B_{max})$ by adjusting the exposure time T and the video stream gain G, and it is necessary to adjust the output power of the main light source 211 by the illumination controller 212. If not, it is known that the current brightness B lies within the target brightness range $(B_{min}, B_{max})$, and the ranging unit 241 is triggered to detect the distance between the end of the endoscope 200 and the target tissue 100. Additionally, the illumination module adjusting unit 243 is under the control of the contact determination unit 242 so that when the contact determination unit 242 is triggered, the illumination module adjusting unit 243 is configured not to adjust the output power of the main light source 211 based on whether the output exposure time T and video stream gain G correspond to the limit values any longer.

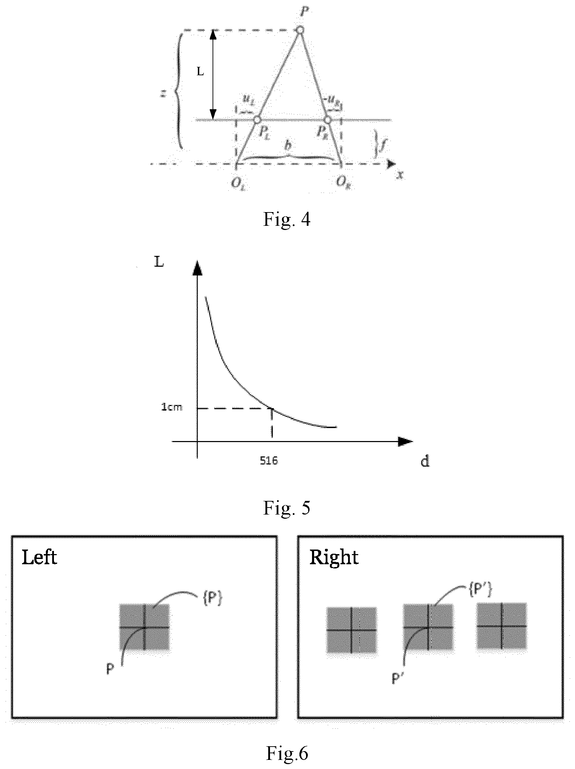

In this embodiment, the ranging unit 241 may acquire the video stream directly from the solid-state photosensitive unit 222 and measure the distance based on an image of the current frame. Alternatively, it may acquire the video stream from the brightness acquisition unit 231 and measure the distance based on an image of the current frame. This embodiment is not limited to any particular method of obtaining the distance between an end of the endoscope 200 and the target tissue 100 by the ranging unit 241. For example, the distance between the end of the endoscope 200 and the target tissue 100 may be obtained using a ranging method utilizing binocular stereoscopic imaging. Fig. 4 schematically illustrates how binocular stereoscopic imaging is utilized to measure the distance from a three-dimensional endoscope to an object (e.g., the target tissue 100) horizontally in front of the endoscope. In Fig. 4, a point on the object to be detected is denoted as P, optical centers of left and right cameras as $O_L$ and $O_R$, respectively, and the distance between the optical centers of the two cameras, i.e., a baseline length, as b. The two cameras observe the feature point P on the target tissue 100 at the same time, and points $P_L$ and $P_R$ can be identified in "left-eye" and "right-eye" images captured in the three-dimensional endoscope. The distances from $P_L$ and $P_R$ respectively to the optical centers $O_L$ and $O_R$ are $\mu_L$ and $|\mu_R|$, and focal lengths of the two cameras are both f.

As shown in Fig. 4, by the principle of similar triangles, we can obtain:

$$\frac{b - \mu_L - |\mu_R|}{b} = \frac{z - f}{z}$$

When defining a parallax as $d = \mu_L + \mu_R$, the distance from the end of the endoscope to the target tissue 100 will be $L = z - f$. Thus, we have $L = f*b/d - f$. Fig. 5 is a schematic diagram showing a relationship between the distance L between the end of the endoscope and the target tissue 100 and the parallax d. As shown in Fig. 5, the greater the parallax is, the closer the end of the endoscope is to the target tissue 100. Moreover, the smaller the parallax is, the more distant the end of the endoscope is from the target tissue 100. For example, when the parallax d is measured to be 516 pixels, the distance L from the end of the endoscope to the target tissue 100 is 1 cm.

More specifically, as shown in Fig. 6, the parallax can be obtained from the "left-eye" and "right-eye" images. Since the three-dimensional endoscope generally has an optical path which is configured so that left and right optical axes have a horizontal common normal, there is only a horizontal translational error or parallax between the "left-eye" and "right-eye" images. The "left-eye" image is scanned and thereby an anchor point P and a region {P} centered thereat like a convolution kernel are selected. Likewise, a region of the same size is selected in the "right-eye" image. Among all such regions in the same row in the "right-eye" image, the one having the smallest sum of the absolute values of the differences between gray scale values of its all pixels and gray scale values of respective pixels in the region {p} is identified as a target region {P'}. Specifically, a region of the same size as the region {P} is selected in the "right-eye" image that has the same horizontal direction as the "left-eye" image. Gray scale values of all the pixels in the region are subtracted from gray scale values of respective pixels in the region {P} of the "left-eye" image, and the sum of the absolute values of the differences between the gray scale values is calculated. This process is repeated on new regions selected by parallel sliding, and the one of all these regions in the "right-eye" image having the smallest sum of absolute values is identified as a target region {P'}. Further, the best matching pixel block P' for the anchor point P in the left image is found, and the parallax d is calculated therefrom. Based on the parallax d, the distance between the end of the endoscope 200 and the target tissue 100 L can be calculated according to the above equation.

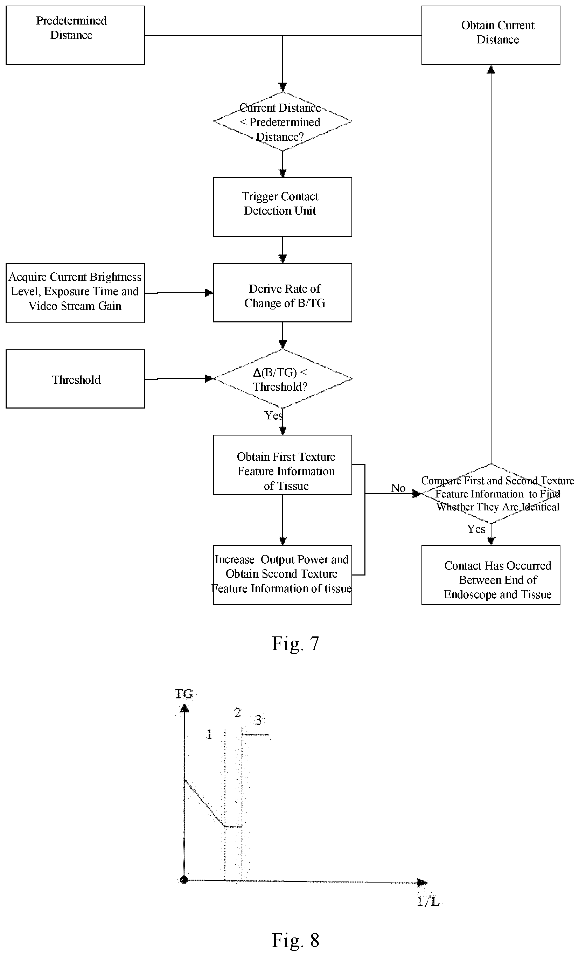

The inventors have found that all the conventional ranging methods based on visual images have some deficiencies. In particular, when an end of an endoscope approaches too close to target tissue 100, these methods are not able to precisely measures the distance between the end of the endoscope and the target tissue 100. For this reason, according to this embodiment, it is determined, based on a relationship among the video stream gain G, the exposure time T and the current brightness B, whether the end of the endoscope 200 is in contact with the target tissue 100. The exposure control unit 232 controls the video stream gain G and the exposure time T, and in the absence of saturated pixels, the current brightness B of the scene of the tissue captured by the solid-state photosensitive unit 222 varies as a linear function of the video stream gain G and the exposure time T. A relationship between the current brightness B, the video stream gain G, the exposure time T and the distance L between the end of the endoscope 200 and the target tissue 100 in a process of the end of the endoscope approaching and coming into contact with the target tissue 100 will be explained below with reference to Figs. 8 to 9.

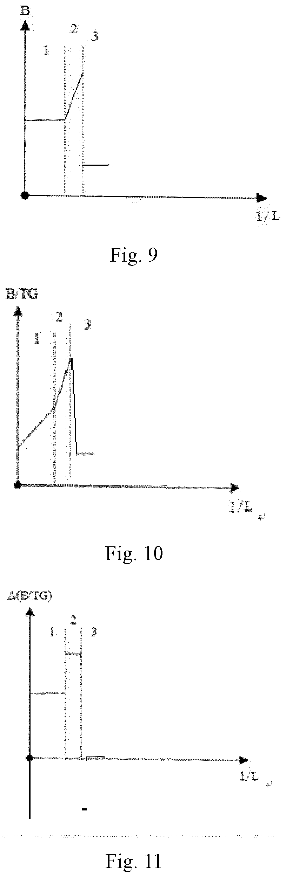

Specifically, assuming the power of the output light is maintained constant, when the end of the endoscope 200 is approaching the target tissue 100 that is being imaged, the current brightness B will continually increase. In order to maintain the current brightness B within the target brightness range ($B_{min}$, $B_{max}$), the exposure control unit 232 will successively reduce the video stream gain G and the exposure time T until the minimum values of them are reached. This process corresponds to the first interval in Figs. 8 and 9. When the end of the endoscope 200 further moves toward the target tissue 100, the current brightness B will continue to increase, despite the fact that the video stream gain G and the exposure time T have been minimized and remain at their respective minimum values. This process corresponds to the second interval in Figs. 8 and 9. The behavior of the current brightness indicates that the end of the endoscope 200 is approaching the target tissue 100, with the brightness rising due to the reflected light. Upon the end of the endoscope 200 coming into contact with the target tissue 100, the current brightness B drops abruptly, and the exposure control unit 232 concurrently outputs the maximum video stream gain G and the maximum exposure time T. At this time, the current brightness B will not change at all even when any of the exposure time T, the video stream gain G and the light source is adjusted. This process corresponds to the third interval in Figs. 8 and 9.

Figs. 10 and 11 further depict variation of the ratio B/TG of the current brightness B to the exposure amount (i.e., the product of the exposure time T and the video stream gain G) and the rate of change $\Delta$(B/TG) of the ratio with the distance from the end of the endoscope to the target tissue 100 when the end of the endoscope is approaching the target tissue 100. As a result of the end of the endoscope moving increasingly closer to the target tissue 100, the main light source will increasingly approach the target tissue 100, illuminating the target tissue 100 with increasing intensity. In order to keep the current brightness B constant, the exposure control unit 232 will increasingly reduce the exposure amount, leading to a rise in the ratio of the current brightness B to the exposure amount TG. At the same time, the rate of change $\Delta(B/TG)$ of the ratio of the current brightness B to the exposure amount TG remains constant. This process corresponds to the first interval in Figs. 10 and 11. When the exposure amount TG is minimized, it remains constant at the minimum value, but the current brightness B continues to rise as the endoscope (and hence the main light source) further approaches the target tissue 100, leading to a different rate of increase of the ratio of the current brightness B to the exposure amount TG from that in the previous interval. This process corresponds to the second interval in Figs. 10 and 11. Upon the end of the endoscope 200 coming into contact with the target tissue 100, the current brightness B of the scene of the tissue drops abruptly below the target brightness range and can never be raised back into the range even when the exposure amount TG is maximized by the exposure control unit 232. As a result, the ratio of the current brightness B to the exposure amount TG is further reduced, and its rate of change $\Delta(B/TG)$ is almost zero. This process corresponds to the third interval in Figs. 10 and 11.

Based on the behavior of the current brightness B, the video stream gain G and the exposure time T across the three intervals in Figs. 8 to 11, it is possible to determine whether the end of the endoscope has come into contact with the target tissue 100. However, due to the translucent nature of human tissue, control of the current brightness of the scene of the tissue and of the exposure amount will be more or less affected by the tissue's translucence. For this reason, misdetermination would be expected when only the above method is relied on for contact detection. In order to overcome this, according to one embodiment of the present invention, a texture feature of the tissue is further extracted in the method, and it is determined whether the end of the endoscope 200 is in contact with the target tissue 100 by further taking into account whether the texture feature changes before and after a change is made in the light source. This imparts increased robustness to the method. Correspondingly, the contact determination unit 242 may be configured to determine whether the end of the endoscope is in contact with the target tissue 100 based on both $\Delta(B/TG)$ and whether the texture feature changes before and after a change is made in the light source. In this case, the contact determination unit 242 may further include a texture feature extraction element 242a and a texture feature comparison element 242b, which are configured for extraction and comparison of the texture feature of the tissue, respectively.

Specifically, when the distance L from the end of the endoscope 200 to the target tissue 100 output from the ranging unit 241 is smaller than the predetermined distance $L_{th}$, the contact determination unit 242 is triggered for contact detection. When triggered, the contact determination unit 242 controls the illumination module adjusting unit 243 in such a way that the power of the output light from the illumination module 210 is no longer adjusted based on information indicating that the current brightness B is not in the predetermined target brightness range $(Bm_{in}, Bm_{ax})$. Preferably, the ranging unit 241 provides a contact alert on a display 300 or the like. As shown in Fig. 7, the ranging unit 241 detects the distance from the end of the endoscope 200 to the target tissue 100, and when the detected distance is smaller than the predetermined distance $L_{th}$ (e.g., 1 cm), the contact detection unit 242 is triggered for contact detection. The contact determination unit 242 acquires the video stream gain G, the exposure time T and the current brightness B of the scene of the tissue from the exposure control unit 232 via the illumination module adjusting unit 243 and derives the rate of change $\Delta(B/TG)$ of B/TG. In an alternative embodiment, the contact determination unit 242 may directly acquire the video stream gain G, the exposure time T and the current brightness B of the scene of the tissue from the exposure control unit 23. When $\Delta(B/TG)$ is smaller than a threshold $\Delta_{th}$, the texture feature extraction element 242a extracts a piece of texture feature information of the target tissue 100 and take it as first texture feature information. After that, the contact detection unit 242 increases the power of the main light source 211 through the illumination module adjusting unit 243, and the texture extraction element 242a again extracts a piece of texture feature information of the target tissue 100 and takes it as second texture feature information. The first texture feature information and the second texture feature information are both input to the texture feature comparison element 242b, which then compares the first texture feature information with the second texture feature information. If no texture feature change is identified from the first texture feature information and the second texture feature information, then it is indicated that the end of the endoscope is in contact with the target tissue 100, and preferably, the contact detection unit 242 provides a contact indication. If a texture feature change is identified from the first texture feature information and the second texture feature information, then it is indicated that the end of the endoscope 200 has suddenly moved away from the target tissue 100 without coming into contact therewith, and preferably, the contact detection unit 242 produces a signal, which triggers the ranging unit 241 to detect the distance again. This embodiment is not limited to any particular texture feature comparison method.

Referring to Fig. 1, according to an embodiment, the endoscope system further includes a video pipeline 250 and a central controller 260. The central controller 260 includes user control logic 261, a user interface 262 and a video processing unit 263. The video stream output from the contact determination module 240 is transmitted to the video pipeline 250, and the video processing unit 263 processes each frame of image in the video pipeline. The images are then transmitted to a surgeon's console and displayed on a display 300 therein. The user control logic 261 is coupled to the endoscope drive module 220 and the contact detection module 240 and is configured to control the endoscope drive module 220 to acquire a scene of the tissue and to control contact detection of the contact detection module 240. Moreover, it provides the user interface 262 with control logic for controlling the video processing unit 263 to process each frame of image in the video pipeline 250.

In one embodiment of the present invention, there is also provided a corresponding method for detecting contact of an end of an endoscope 200 with target tissue 100, which includes:

providing output light for illuminating the target tissue 100 to generate reflected light;
acquiring a scene of the target tissue 100 through capturing the reflected light based on an exposure time T and a video stream gain G and outputting the scene in the form of a video stream;
acquiring a current brightness B of the video stream and tuning the current brightness B of the video stream into a predetermined target brightness range by adjusting the video stream gain G and the exposure time T; and
in the event of the current brightness B of the video stream lying in the target brightness range and of a distance from the end of the endoscope 200 to the target tissue 100 being smaller than a predetermined distance $L_{th}$, determining whether a rate of change of a ratio B/TG of the current brightness to an exposure amount is smaller than a threshold $\Delta_{th}$, and if so, then
extracting texture feature information of the target tissue 100 as first texture feature information;
increasing a power of the output light and then extracting texture feature information of the target tissue 100 as second texture feature information; and
comparing the first texture feature information and the second texture feature information, and if no texture feature change is identified, determining that the end of the endoscope 200 is in contact with the target tissue.

Optionally, the distance from the end of the endoscope 200 to the target tissue 100 may be obtained by a ranging method based on binocular stereoscopic imaging.

Optionally, when the current brightness B is lower than a lower limit $B_{min}$ of the target brightness range, the current brightness B may be adjusted by increasing the exposure amount.

Optionally, it may be determined whether a maximum exposure time $T_{max}$ and a minimum video stream gain $G_{min}$ satisfy an exposure amount requirement of the target brightness range.

If the requirement is satisfied, on the basis of the minimum video stream gain $G_{min}$, the exposure time may be adjusted to tune the current brightness B into the target brightness range.

If the requirement is not satisfied, the video stream gain may be increased, and it may be determined whether the maximum exposure time $T_{max}$ and a maximum video stream gain $G_{max}$ satisfy the exposure amount requirement of the target brightness range.

If the requirement is satisfied, on the basis of the maximum exposure time $T_{max}$, the video stream gain may be adjusted to tune the current brightness B of the video stream into the target brightness range.

Optionally, before it is determined whether the rate of change $\Delta(B/TG)$ of the ratio B/TG of the current brightness to the exposure amount is smaller than the threshold $\Delta_{th}$, if the maximum exposure time $T_{max}$ and the maximum video stream gain $G_{max}$ do not satisfy the exposure amount requirement of the target brightness range, the power of the output light may be adjusted.

Optionally, if the current brightness B is higher than an upper limit $B_{max}$ of the target brightness range, the current brightness B may be adjusted by reducing the exposure amount.

Optionally, it may be determined whether the maximum exposure time $T_{max}$ and the minimum video stream gain $G_{min}$ satisfy the exposure amount requirement of the target brightness range.

If the requirement is satisfied, on the basis of the maximum exposure time $T_{max}$, the video stream gain may be adjusted to tune the current brightness B of the video stream into the target brightness range.

If the requirement is not satisfied, the exposure time may be reduced, and it may be determined whether a minimum exposure time $T_{min}$ and the minimum video stream gain $G_{min}$ satisfy the exposure amount requirement of the target brightness range.

If the requirement is satisfied, on the basis of the minimum video stream gain $G_{min}$, the exposure time may be adjusted to tune the current brightness B of the video stream into the target brightness range.

Optionally, before it is determined whether the rate of change of the ratio of the current brightness to the exposure amount is smaller than the threshold, if the minimum exposure time $T_{min}$ and the minimum video stream gain $G_{min}$ do not satisfy the exposure amount requirement of the target brightness range, the power of the output light may be adjusted.

In summary, the present invention provides an endoscope system and a method for detecting contact of an end of an endoscope with target tissue, in which a video stream gain and an exposure time can be adjusted to maintain a current brightness of a video stream within a target brightness range, and the distance from the end of the endoscope to the target tissue can be measured. When the distance from the end of the endoscope to the target tissue is smaller than a predetermined distance, a contact alert is provided, and contact determination is triggered. During detection of a rate of change of a ratio of the current brightness to an exposure amount is smaller than a threshold, texture feature information of the target tissue may be extracted respectively before and after a power of output light is increased and compared to determine whether the end of the endoscope is in contact with the target tissue. By determining whether the end of the endoscope is in contact with the target tissue through comparing the texture feature information of the target tissue extracted respectively before and after the power of the output light is increased to find whether there is a texture feature change, the endoscope system of the present invention can provide a more accurate contact indication and effectively avoid the endoscope from causing damage to the target tissue during its examination of the tissue.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. An endoscope system, comprising an endoscope, an illumination module, an endoscope drive module, an endoscope control module and a contact detection module,

    wherein the illumination module is configured to provide an output light for illuminating a target tissue and to generate a reflected light,
    wherein the endoscope drive module is configured to acquire a scene of the target tissue through capturing the reflected light based on an exposure time and a video stream gain and to output the scene in a form of a video stream,
    wherein the endoscope control module is communicatively connected to the endoscope drive module, wherein the endoscope control module is configured to acquire a current brightness of the video stream from the video stream, and wherein a video stream gain and an exposure time are adjusted, such that the current brightness of the video stream is within a target brightness range; wherein the contact detection module is communicatively connected to each of the endoscope control module and the illumination module, the contact detection module comprising a contact determination unit, wherein when the current brightness of the video stream lies within the target brightness range and a distance from an end of the endoscope to the target tissue is smaller than a predetermined distance, the contact determination unit is configured to determine a relationship between a rate of change of a ratio of the current brightness to an exposure amount and a threshold, and wherein when the rate of change brightness is smaller than the threshold, the contact determination unit is configured to control the illumination module to increase the power of the output light, and then extract and compare texture feature information of the target tissue respectively before and after the power of the output light is increased so as to determine whether the end of the endoscope is in contact with the target tissue, and to provide a contact indication.

2. The endoscope system according to claim 1, wherein the contact detection module further comprises a ranging unit configured to detect the distance from the end of the endoscope to the target tissue.

3. The endoscope system according to claim 2, wherein the ranging unit provides a contact alert upon detection of the distance from the end of the endoscope to the target tissue being smaller than the predetermined distance.

4. The endoscope system according to claim 2, wherein the endoscope is a three-dimensional endoscope and wherein the ranging unit obtains the distance from the end of the endoscope to the target tissue using a ranging method based on a binocular stereoscopic imaging.

5. The endoscope system according to claim 1, wherein the endoscope control module comprises a brightness acquisition unit configured to acquire the current brightness of the video stream.

6. The endoscope system according to claim 5, wherein in case of the video stream being a YUV-coded stream, the brightness acquisition unit is configured to take an averaged or a weighted value of Y values of all or some pixels in an image of a current frame as the current brightness.

7. The endoscope system according to claim 5, wherein: in case of the video stream being a RGB-coded stream, the brightness acquisition unit is configured to derive a brightness of each pixel in an image of a current frame from a RGB value of the pixel and then take an averaged or a weighted value of the brightness of all or some of the pixels in the image as the current brightness; or the brightness acquisition unit is configured to take an averaged or a weighted value of G values of all or some of the pixels in the image of the current frame as the current brightness.

8. The endoscope system according to claim 1, wherein the endoscope control module comprises an exposure control unit configured to determine whether the current brightness of the video stream is within the target brightness range and, when the current brightness of the video stream is not within the target brightness range, the exposure control unit is configured to adjust the video stream gain and the exposure time to enable the current brightness of the video stream to be within the target brightness range.

9. The endoscope system according to claim 8, wherein when the current brightness is lower than a lower limit of the target brightness range, the exposure control unit adjusts the current brightness by increasing the exposure amount.

10. The endoscope system according to claim 9, wherein the exposure control unit determines whether a maximum exposure time and a minimum video stream gain can satisfy an exposure amount requirement of the target brightness range, and

if the requirement is satisfied, on a basis of the minimum video stream gain, adjusting the exposure time to enable the current brightness to be within the target brightness range; or
if the requirement is not satisfied, increasing the video stream gain and determining whether the maximum exposure time and a maximum video stream gain satisfy the exposure amount requirement of the target brightness range, and if the requirement is satisfied, on a basis of the maximum exposure time, adjusting the video stream gain to enable the current brightness of the video stream to be within the target brightness range.

11. The endoscope system according to claim 8, wherein when the current brightness is higher than an upper limit of the target brightness range, the exposure control unit adjusts the current brightness by reducing the exposure amount.

12. The endoscope system according to claim 11, wherein the exposure control unit determines whether a maximum exposure time and a minimum video stream gain can satisfy an exposure amount requirement of the target brightness range,

if the requirement is satisfied, on a basis of the maximum exposure time, adjusting the video stream gain to enable the current brightness of the video stream to be within the target brightness range; or
if the requirement is not satisfied, reducing the exposure time and determining whether a minimum exposure time and the minimum video stream gain can satisfy the exposure amount requirement of the target brightness range, and if the requirement is satisfied, on a basis of the minimum video stream gain, adjusting the exposure time to enable the current brightness of the video stream to be within the target brightness range.

13. The endoscope system according to claim 8, wherein when the exposure control unit is not able to adjust the video stream gain and the exposure time to enable the current brightness of the video stream to be within the target brightness range, the illumination module is controlled to adjust the power of the output light.

14. The endoscope system according to claim 13, wherein the contact detection module further comprises an illumination module adjusting unit communicatively connected to each of the illumination module and the exposure control unit, wherein the illumination module adjusting unit is configured to determine whether the current brightness is within the target brightness range based on the video stream gain and the exposure time output from the exposure control unit, and to adjust the power of the output light through the illumination module.

15. The endoscope system according to claim 14, wherein the illumination module adjusting unit is also communicatively connected to the contact determination unit, further configured to, under a control of the contact determination unit, adjust the power of the output light through the illumination module, and wherein while the contact determination unit is determining whether the end of the endoscope is in contact with the target tissue, the illumination module adjusting unit is controlled not to be instructed by the exposure control unit to adjust the power of the output light any more.

16. The endoscope system according to claim 1, wherein the endoscope drive module comprises a solid-state photosensitive unit and a scene drive unit, wherein the solid-state photosensitive unit is configured to receive the reflected light that carries information of the scene of the target tissue, and to perform an optical-to-electronic conversion on the information of the scene of the target tissue and to output the information of the scene of the target tissue as the video stream, and wherein the scene drive unit is configured to control the solid-state photosensitive unit based on the video stream gain and the exposure time received from the endoscope control module.

17. The endoscope system according to claim 1, wherein the contact detection module further comprises an illumination module adjusting unit communicatively connected to each of the contact determination unit and the exposure control unit, wherein the illumination module adjusting unit is configured to receive the video stream gain, the exposure time and the current brightness output from the exposure control unit, and wherein the contact determination unit is configured to determine the rate of change of the ratio of the current brightness to the exposure amount based on the video stream gain, the exposure time and the current brightness received by the illumination module adjusting unit.

18. The endoscope system according to claim 1, wherein the contact determination unit comprises a texture feature extraction element and a texture feature comparison element, which are configured to extract and compare texture features of the target tissue, respectively.

19. The endoscope system according to claim 1, further comprising a video pipeline and a central controller, wherein the central controller comprises a video processing unit, wherein the video stream output from the contact detection module is transmitted to the video pipeline, and wherein the video processing unit processes the video stream in the video pipeline and transmits the processed video stream to a display device for display thereon.

20. A method for detecting a contact of an end of an endoscope with a tissue, comprising:

   providing an output light for illuminating a target tissue to generate a reflected light;
   acquiring a scene of the target tissue through capturing the reflected light based on an exposure time and a video stream gain, and outputting the scene in a form of a video stream;
   acquiring a current brightness of the video stream, and adjusting the video stream gain and the exposure time to enable the current brightness of the video stream to be within a target brightness range; and
   when the current brightness of the video stream is within the target brightness range and a distance from an end of the endoscope to the target tissue is smaller than a predetermined distance, determining whether a rate of change of a ratio of the current brightness to an exposure amount is smaller than a threshold, if so, then
   extracting a texture feature information of the target tissue as a first texture feature information;
   increasing a power of the output light and then extracting a texture feature information of the target tissue as a second texture feature information; and
   comparing the first texture feature information and the second texture feature information, and if no texture feature change is identified, determining that a contact of the end of the endoscope with the target tissue has occurred.

21. The method according to claim 20, wherein the distance from the end of the endoscope to the target tissue is obtained by a ranging method based on binocular stereoscopic imaging.

22. The method according to claim 20, wherein when the current brightness is lower than a lower limit of the target brightness range, the current brightness is adjusted by increasing the exposure amount.

23. The method according to claim 20, further comprising determining whether a maximum exposure time and a minimum video stream gain satisfy an exposure amount requirement of the target brightness range, and

   if the requirement is satisfied, on a basis of the minimum video stream gain, the exposure time is adjusted to enable the current brightness to be within the target brightness range; or
   if the requirement is not satisfied, the video stream gain is increased, followed by determining whether the maximum exposure time and a maximum video stream gain satisfy the exposure amount requirement of the target brightness range, and if the requirement is satisfied, on a basis of the maximum exposure time, the video stream gain is adjusted to enable the current brightness of the video stream to be within the target brightness range.

24. The method according to claim 23, wherein before determining whether the rate of change of the ratio of the current brightness to the exposure amount is smaller than the threshold, if the maximum exposure time and the maximum video stream gain do not satisfy the exposure amount requirement of the target brightness range, then the power of the output light is adjusted.

25. The method according to claim 20, wherein if the current brightness is higher than an upper limit of the target brightness range, the current brightness is adjusted by reducing the exposure amount.

26. The method according to claim 25, further comprising determining whether a maximum exposure time and a minimum video stream gain satisfy an exposure amount requirement of the target brightness range, and

   if the requirement is satisfied, on a basis of the maximum exposure time, the video stream gain is adjusted to enable the current brightness to be within the target brightness range; or
   if the requirement is not satisfied, the exposure time is reduced, followed by determining whether a minimum exposure time and the minimum video stream gain satisfy the exposure amount requirement of the target brightness range, and if the requirement is satisfied, on a basis of the minimum video stream gain, the exposure time is adjusted to enable the current brightness of the video stream to be within the target brightness range.

27. The method according to claim 26, wherein before determining whether the rate of change of the ratio of the current brightness to the exposure amount is smaller than the threshold, if the minimum exposure time and the minimum video stream gain do not satisfy the exposure amount requirement of the target brightness range, the power of the output light is adjusted.

28. The method according to claim 20, wherein when the distance from the end of the endoscope to the target tissue is smaller than the predetermined distance, a contact alert is provided.

Fig. 1

Fig. 2

Predetermined Target Brightness Range $(B_{min}, B_{max})$

Acquire Current Brightness Level B

B > $B_{max}$? — No / Yes

Reduce Exposure Amount

Increase Exposure Amount

B < $B_{min}$? — Yes / No

Maximum Exposure Time and Minimum Video Stream Gain Satisfy Exposure Amount Requirement? — No / Yes

Minimum Exposure Time and Minimum Video Stream Gain Satisfy Exposure Amount Requirement? — Yes / No

Maximum Exposure Time and Minimum Video Stream Gain Satisfy Exposure Amount Requirement? — Yes / No

Maximum Exposure Time and Maximum Video Stream Gain Satisfy Exposure Amount Requirement? — Yes / No

Maintain Minimum Video Stream Gain and Increase Exposure Time to Meet Exposure Amount Requirement

Maintain Maximum Exposure Time and Adjust Video Stream Gain to Meet Exposure Amount Requirement

Maintain Maximum Exposure Time and Adjust Video Stream Gain to Meet Exposure Amount Requirement

Maintain Minimum Video Stream Gain and Adjust Exposure Time to Meet Exposure Amount Requirement

Output Minimum Video Stream Gain and Exposure Time

Output Maximum Exposure Time and Maximum Video Stream Gain

Output Maximum Exposure Time and Video Stream Gain

Output Current Exposure Time and Video Stream Gain

Output Maximum Exposure Time and Video Stream Gain

Output Minimum Video Stream Gain and Minimum Exposure Time

Output Minimum Video Stream Gain and Exposure Time

Fig. 3

EP 4 202 526 A1

18

Fig. 4

Fig. 5

Fig.6

Fig. 7

Fig. 8

Fig. 9

Fig. 10

Fig. 11

## INTERNATIONAL SEARCH REPORT

| International application No. |
|---|
| **PCT/CN2021/109859** |

| **A.** | **CLASSIFICATION OF SUBJECT MATTER** |
|---|---|

G02B 23/24(2006.01)i;  A61B 1/00(2006.01)i

According to International Patent Classification (IPC) or to both national classification and IPC

| **B.** | **FIELDS SEARCHED** |
|---|---|

Minimum documentation searched (classification system followed by classification symbols)

G02B; A61B

Documentation searched other than minimum documentation to the extent that such documents are included in the fields searched

Electronic data base consulted during the international search (name of data base and, where practicable, search terms used)

CNABS; CNTXT; CNKI; VEN; JPABS; USTXT; WOTXT; EPTXT: 内窥镜, 驱动, 控制, 接触, 组织, 反射, 视频, 增益, 亮度, endoscope, drive, control, contact, tissue, reflect+, video, gain, brightness

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 111948798 A (MICROPORT (SHANGHAI) MEDICAL ROBOT CO., LTD.) 17 November 2020 (2020-11-17)<br>        description, paragraphs [0004]-[0126] | 1-28 |
| Y | CN 106456276 A (INTUITIVE SURGICAL, INC.) 22 February 2017 (2017-02-22)<br>        description, paragraphs [0033]-[0164], figures 1A-7 | 1-28 |
| Y | CN 105228501 A (OLYMPUS CORPORATION) 06 January 2016 (2016-01-06)<br>        description, paragraphs [0029]-[0138], and figures 1-11 | 1-28 |
| Y | CN 203153700 U (GUANGZHOU BAODAN MEDICAL INSTRUMENT TECHNOLOGY CO., LTD.) 28 August 2013 (2013-08-28)<br>        description, paragraphs [0004]-[0019], figures 1, 2 | 1-28 |
| A | CN 109673081 A (SHANGHAI AOHUA PHOTOELECTRICITY ENDOSCOPE CO., LTD.) 23 April 2019 (2019-04-23)<br>        entire document | 1-28 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 September 2021** | **20 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/109859**

| Patent document cited in search report | | | Publication date (day/month/year) | Patent family member(s) | | | Publication date (day/month/year) |
|---|---|---|---|---|---|---|---|
| CN | 111948798 | A | 17 November 2020 | None | | | |
| CN | 106456276 | A | 22 February 2017 | JP | 2017510348 | A | 13 April 2017 |
| | | | | KR | 20160134739 | A | 23 November 2016 |
| | | | | EP | 3119318 | A1 | 25 January 2017 |
| | | | | US | 10512512 | B2 | 24 December 2019 |
| | | | | JP | 6707033 | B2 | 10 June 2020 |
| | | | | EP | 3119318 | A4 | 13 December 2017 |
| | | | | US | 2020078108 | A1 | 12 March 2020 |
| | | | | CN | 106456276 | B | 10 December 2019 |
| | | | | US | 2017095297 | A1 | 06 April 2017 |
| | | | | WO | 2015142799 | A1 | 24 September 2015 |
| | | | | JP | 2020171710 | A | 22 October 2020 |
| | | | | CN | 110772205 | A | 11 February 2020 |
| CN | 105228501 | A | 06 January 2016 | JP | 6265627 | B2 | 24 January 2018 |
| | | | | EP | 3001941 | A4 | 25 January 2017 |
| | | | | EP | 3001941 | A1 | 06 April 2016 |
| | | | | WO | 2014188740 | A1 | 27 November 2014 |
| | | | | US | 2016038004 | A1 | 11 February 2016 |
| | | | | JP | 2014226341 | A | 08 December 2014 |
| CN | 203153700 | U | 28 August 2013 | None | | | |
| CN | 109673081 | A | 23 April 2019 | None | | | |

Form PCT/ISA/210 (patent family annex) (January 2015)